# EUROPEAN PATENT APPLICATION

(11) **EP 4 241 828 A1**
(43) Date of publication of application: **13.09.2023**
(21) Application number: 22161415.9
(22) Date of filing: 10.03.2022
(51) Int. Cl.: A61M 39/20, A61M 39/22, A61M 39/18, A61M 39/26

(54) **CLOSING ELEMENT FOR A FLUID LINE**

(71) Applicant: Fresenius Medical Care Deutschland GmbH, 61352 Bad Homburg (DE)
(72) Inventor: DUMONT-D'AYOT, François, 61352 Bad Homburg (DE); LAFFAY, Philippe, 61352 Bad Homburg (DE)
(74) Representative: Herrmann, Uwe

(57) **Abstract**

The present invention relates to a closing element for a fluid line, preferably a fluid line of a concentrate container, comprising a main body configured to at least partially receive the fluid line, a closing portion configured to be arranged on an open portion of the fluid line to close the fluid line, wherein the closing portion comprises a plurality of plates arranged around a common center point and each separated from another over an intended breaking point, an a pressure receiving portion configured to receive pressure applied by a pressure exertion element, preferably a connector element to be fluidically connected to the fluid line by breaking the intended breaking point, wherein the pressure receiving portion is formed by that part of the plurality of plates projecting outwards from the closing portion or the main body.

## Description

The present invention relates to a closing element for a fluid line, e.g. of a concentrate container for medical purposes, a connector comprising a fluid line closed by such a closing element and a system comprising a concentrate container having such a connector that is closed by such a closing element and a connector of a blood treatment device or dosing unit thereof.

In the process of blood treatment, e.g. a dialysis treatment, the required dialysis solution is often produced from dry concentrate that is mixed with pure water. For this purpose, containers containing a concentrate, e.g. bicarbonate or an acidic concentrate, are fluidically connected to the blood treatment device or a dosing unit thereof to allow mixing of a solution of a desired concentrate concentration.

Prior to connection of the container to the blood treatment device or dosing unit, the concentrate containers are sealed for storage. Prior to fluidically connecting the container to the blood treatment device or dosing unit, conventionally a seal is manually removed from the fluid lines of the concentrate container to open the fluid lines for the connection to corresponding connector elements of the blood treatment device. Thus, conventionally there is a significant risk of contamination due to extensive manual handling of the concentrate container. In addition to that, the film conventionally used for sealing the containers may easily be dislodged during storage of the container leading to a potentially contaminated and thus unusable container.

Another common approach to close medical containers which have to be fluidly connected to another fluid line for delivering the content to a treatment machine or a patient are closures with intended breaking edges. These closures do not have to be removed before use, but the connection is established by a part, for example a spike, which is introduced into the closing element having an intended breaking point. This has the disadvantage, that the part for establishing the connections firstly touches the outer surface of the closure, which can be contaminated.

It is an object of the present invention to alleviate or even overcome the problems of the prior art.

In particular, it is an object of the present invention to introduce a system that can be opened from the outside while being sure there is no contact between the outside and the product content.

Thereby a safe, reliable and hygienic means for fluidically closing a concentrate container, especially in a case in that the concentrate container is not directly manually opened but opened by the blood treatment device or dosing unit thereof, e.g. by manual movement of a connector element of the blood treatment device or dosing unit thereof, prior to connection thereto is provided. Especially in case of a manual connection of the concentrate container to the blood treatment device or dosing unit thereof, it is important to ensure that only a minimal amount of force is required for connecting. This reduces the strain on personnel that in daily practice has to perform such a connection day-to-day with a high frequency.

This object is achieved by the invention as recited in the independent claims. Further embodiments of the invention are the subject-matter of the dependent claims.

A first aspect of the invention relates to a closing element for a fluid line, in particular a fluid line of a concentrate container, comprising:
a main body configured to at least partially receive the fluid line,
a closing portion configured to be arranged on an open portion of the fluid line to close the fluid line, wherein the closing portion comprises a plurality of plates arranged around a common center point and each separated from another over an intended breaking point, and
a pressure receiving portion configured to receive pressure applied by a pressure exertion element, in particular a connector element to be fluidically connected to the fluid line by breaking the intended breaking point, wherein
the pressure receiving portion is formed by that part of the plurality of plates projecting outwards from the closing portion or the main body.

The closing portion may be configured to at least partially close an open portion of the fluid line.

The main body may comprise a hollow body having a circumferential wall with an open end. The closing portion may be movably joined to the circumferential wall at the open end of the main body. The main body may be arranged to receive the fluid line in the hollow body. Movably joined may have the meaning that the position of closing portion may be changed relative to the main body without detaching the closing portion from the main body.

In the sense of the present description, the expression pressure receiving portion may be equivalent to the meaning of a force receiving portion as it is only crucial that force is applied to the pressure receiving portion to open the closing element. In other words, it is obvious that the expressions pressure and force may be considered equivalent with respect to the opening of the closing element.

Each plate may be arranged on the main body so that a length of a part of each plate projecting outwards from the main body is at least 80% of a length of a part of each plate projecting inwards from the main body. In particular, the length of the part of each plate projecting outwards from the main body may be equal to the length of the part of each plate projecting inwards from the main body. The length of the part of each plate projecting outwards from the main body may also be larger than the length of the part of each plate projecting inwards from the main body.

The part of each plate projecting outwards from the main body may be regarded as a part forming the pressure receiving portion and the part of each plate projecting inwards from the main body may be regarded as a part forming the closing portion. The part of each plate in that the plate is supported by the main body may be regarded as a support portion that separates the pressure receiving portion from the closing portion of each plate.

When measuring the length of the part of each plate projecting outwards from the main body and the length of the part of each plate projecting inwards from the main body, each measurement starts in the middle of the support portion and ends at the inner or outer edge of the plate, respectively. Thus, the width of the support portion and thus the thickness of a wall of the main body does not affect the relative relationship of the length of the part of each plate projecting outwards from the main body and the length of the part of each plate projecting inwards from the main body.

The pressure receiving portion and the closing portion may be regarded as functional portions of a plate, wherein the plate may be formed as one piece or may comprise several pieces. Thus, the limits of the pressure receiving portion and the closing portion of each plate may not necessarily coincide with the limits of the pieces forming the plate.

Through a careful selection of the dimensions of the closing element, the force for opening the closing element may be minimized and may be as low as 46 Newton.

Each plate may have a thickness of between 1,5 mm and 3 mm, or between 2 mm and 2,5 mm. The outer edge of each plate may have a length of between 3,5 mm to 5,5 mm, or between 4 mm to 5 mm, in particular between 4, 5 mm and 5 mm. The wall or the walls of the main body may have a thickness of between 0,7 mm and 0,9 mm, in particular between 0,8 and 0,85 mm.

According to an embodiment of the invention, a length from an outer edge of a first plate of the plurality of plates to an outer edge of a second plate of the plurality of plates arranged opposed to the first plate lies in the range of between 10 mm and 20 mm, in particular between 12 mm and 18 mm, in particular between 16 mm and 17 mm.

A width of an intended breaking point separating a first plate of the plurality of plates from a second plate of the plurality of plates may lie in the range of between 0,1 mm and 0,5 mm, in particular between 0,2 mm and 0,4 mm, in particular between 0,25 mm and 0,35 mm.

A strength of the intended breaking point separating a first plate of the plurality of plates from a second plate of the plurality of plates may lie in the range of between 0,1 mm and 0,3 mm, in particular between 0,15 mm and 0,25 mm, in particular between 0,2 mm and 0,25 mm. The term strength has a meaning of a thickness of the material.

Intended breaking points may be provided between each adjacent plates of the plurality of plates. All intended breaking points may have the same shape or may differ in shape, in particular every second breaking point may have the same shape but differ from the directly adjacent breaking point.

The position of the intended breaking point or points may be located - relative along the thickness of the plate - at the upper end of the plate or plates or at the lower end of the plate or plates, the lower end being the end of the plate closer to the main body of the closing element, or at the center of the plate or plates.

The term intended breaking point is equivalent to the term predetermined breaking point. Thus, the term intended does not refer to a human perception but to a technical feature as it defines where the material is broken upon exertion of the pressure.

According to an embodiment of the invention, the pressure receiving portion comprises a plurality of protrusions arranged along the outer circumference of the closing element, for example at the pressure receiving portion thereof, wherein the protrusions may be separated from another and / or arranged in equal intervals along the outer circumference. Each protrusion may also be connected to the adjacent protrusions, in particular by further breaking points that may be broken upon exertion of the pressure to open the closing element.

Each protrusion of the plurality of protrusions may be arranged adjacent to an outer edge of a plate of the plurality of plates so that pressure applied to each protrusion is selectively transferred to the plate the protrusion is arranged on, wherein a length from a protrusion of a first plate of the plurality of plates to protrusion of a second plate of the plurality of plates arranged opposed to the first plate lies in the range of between 10 mm and 20 mm, in particular between 12 mm and 18 mm, in particular between 16 mm and 16,6 mm.

According to an embodiment of the invention, the pressure receiving portion and / or at least one protrusion thereof at least partially defines a distal end face of the closing element in the insertion direction of the fluid line into the closing element.

According to another embodiment of the invention, the main body has a hexagonal form, wherein a distance between a first outer side surface of the hexagon and a second outer side surface of the hexagon arranged opposed to the first outer side surface lies in the range of between 6 mm and 10 mm, in particular be-tween 7,5 mm and 9,5 mm, in particular between 8 mm and 9 mm.

The main body with the hexagonal form may have a distance between a first inner side surface of the hexagon and a second inner side surface of the hexagon arranged opposed to the first inner side surface in the range of between 4 mm and 9 mm, in particular between 5,5 mm and 8,5 mm, in particular between 6,5 mm and 7,5 mm. The width of the wall between the inner surface side and the outer surface side may be equal or larger than 1 mm, in particular equal or larger than 2 mm.

It has been shown that if at a connection point between the closing portion comprising the intended breaking point and the main body of the closing element the material is selectively weakened, in particular by providing at least one annular recess running along the circumference of the main body, to facilitate breaking of the intended breaking point due to pressure being applied to the pressure receiving portion, the required pressure is reduced, in particular wherein a width of the annular recess lies in the range of between 0,3 mm and 0,6 mm, in particular in the range between 0, 35 mm and 0,55 mm, in particular between 0,4 mm and 0,5 mm. Thus, the main body material may be weakened in some areas in comparison to other areas, in particular the weakened area may be located adjacent to the closing portion and/or may have a recess running along the circumference of the main body.

The main body may be bendable, in particular the main body may be bendable in the area of weakening and/or recess. Upon opening of the closing element by tilting the closing portion the main body may be bent on an upper rim joint to the plates of the closing portion without being separated or detached from the plates. Bendable here may have the meaning of a flexibility and the flexibility of the main body may be larger in a first portion of the main body being connected to the closing portion than in a second portion being further distant from the closing portion. In other words, the main body may be shaped to change its shape upon exertion of the opening pressure to the opening portion, in particular in an area connected to the opening portion whereas another part of the main body is configured to not change its shape upon exertion of the pressure.

According to an embodiment, the closing element is at least partially or completely manufactured from polymer material, in particular from linear low density polyethylene and / or high density polyethylene.

Another aspect of the invention relates to a connector, in particular a connector of a concentrate container or other disposable, comprising at least one fluid line providing a flow path through the connector, wherein a closing element according to the invention is arranged on at least one fluid line of the connector to close the fluid line. The closing effect may be a closing effect to block fluid from exiting and/or entering the connector.

According to an embodiment, the fluid line comprises an inner lumen and/or an outer lumen, wherein the inner lumen may have a hexagonal form and the closing element is arranged on an end face of the inner lumen, wherein the end face of the inner lumen may comprise a champfer, in particular at its leading edge.

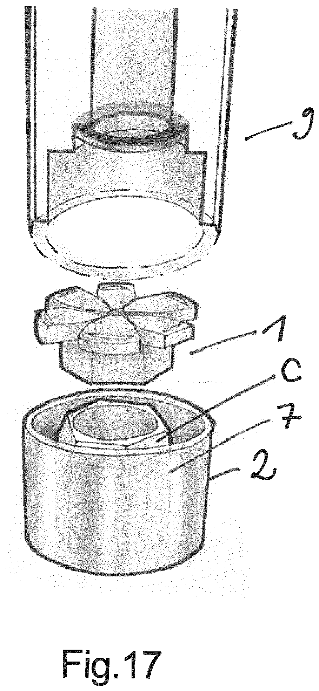

In this regard, the term lumen may not only refer to a hollow space, e.g. a hollow flow path for e.g. conducting fluid but also may refer to the walls defining the hollow flow path. In other words, the term inner lumen may be inner wall and the term outer lumen may be an outer wall wherein the lumen may be the hollow space between the two walls. In an embodiment in that the inner lumen has a hexagonal form, for example, the contour of an outer circumferential surface of the wall defining the inner limitation of the lumen may be hexagonal. The inner lumen itself may form a wall of another lumen, i.e. hollow space, located and surrounded by the inner lumen. The contour of an inner circumferential surface of the inner lumen forming the other lumen may also have a hexagonal form leading to a substantially constant wall thickness along the entire circumference of the wall or may have a different geometry, e.g. a circular form as shown in Fig. 17, thus leading to differing wall thicknesses along the circumference of the wall. It is noted that the outer lumen 2 as it is shown for example in Fig. 17 is an optional feature of the connector there may still be an inner lumen present in all embodiments even if there is no outer lumen present. This is the case as the inner lumen defines the fluid path of the connector to be connected to fluid path of the machine and the closing element may be arranged on the inner lumen.

In other words, in an embodiment in that the inner lumen has a hexagonal form, the actual hollow space and / or the wall limiting the hollow space, in particular an outer circumferential surface thereof, may have a hexagonal form. The same applies to any other desirably geometry chosen instead of a hexagonal form.

Similarly, in an embodiment in that the end face of the inner lumen comprises a champfer, the champfer may be present at the wall defining the inner lumen, as depicted for example in Fig. 17.

The champfer of the leading edge of the inner lumen may be present on a radially outward side of the inner lumen and the champfer may comprise a tapered portion inclined with an angle of between 35 ° to 65°, in particular between 40° and 50°, in particular between 42° and 47°.

Another aspect of the invention relates to a system comprising a closing element according to the invention, a connector according to the invention and a pressure exertion element, in particular a connector element of a blood treatment device or a dosing unit thereof, wherein the pressure exertion element is configured to be fluidically connected to the fluid line of the connector by breaking the intended breaking point of the closing element, in particular by a relative movement of the pressure exertion element and the connector.

The intended breaking point may be an area of weakened material, e.g. a local thinning of the material. The intended breaking point may have the shape of a line or multiple lines.

The fluid line may be any tube or hose suitable for conducting fluid. The fluid line may in particular be a formstable hose which may be made of a hard plastic material.

The term tube is equivalent to the term hose. If in this description any of the two terms tube or hose is used it is meant to be exchangeable by the other term.

The at least one protrusion of the pressure receiving portion projects outwards from the closing portion, the pressure receiving portion or the main body of the closing element. In other words, the protrusion projects away from at least a part of an outer surface of the closing portion, the pressure receiving portion or the main body of the closing element. For example, the protrusion may be regarded as a sub section of the pressure receiving portion that bulges outwards relative from another sub section of the pressure receiving portion. The same may apply if the projection projects outwards from the closing portion or the main body of the closing element.

For example, as the protrusion projects outwards from the closing portion, pressure may be exerted onto the protrusion by a pressure exertion element without the pressure exertion element contacting an outer surface of the closing portion. Thus, it is ensured that the closing portion does never enter and / or completely remains outside of the flow path established upon opening of the closing element.

According to an embodiment of the invention, the pressure receiving portion comprises an annular protrusion running at least along a part of or along the entire length of the outer circumference of the closing element.

According to another embodiment of the invention, the pressure receiving portion comprises a plurality of protrusions arranged along the outer circumference of the closing element, for example at the pressure receiving portion and / or closing portion thereof, wherein the protrusions may be separate from another and / or arranged in equal intervals along the outer circumference.

In an embodiment, the closing portion is formed by a plurality of plates each having the shape of a circle section that are arranged around a center point of the closing portion similar to petals of a flower.

Each plate of the plurality of plates may be separated from the adjacent plate by an intended breaking point, e.g. a line of local thinning of the material. In an embodiment, the closing portion comprises six plates ("petals") and further the main body has a hexagonal base shape. In this embodiment for each of the individual plates the individual plates may be arranged on one side of the six sides of the hexagonal base shape of the main body.

Each protrusion of the plurality of protrusions may be arranged on a plate forming the closing portion so that pressure applied to each protrusion is selectively transferred to the plate the protrusion is arranged on. The pressure exertion element presses onto the plates, in particular onto the radially outward section of each plate, thus causing each plate to tilt like a lever, so that the radially outward section of each plate and the radially inward section of each plate are moved in opposing directions. The tilting movement of the plates strains the intended breaking point until it breaks. The breaking of the intended breaking point may be further supported by the upper edge of the fluid line closed by the closing element pressing against the plates of the closing portion from the inside of the main body of the closing element. In conjunction with the arrangement of the plates forming the closing portion and the provision of an intended breaking point between each two adjacent plates of the closing portion, the force required to open the closing element can be reduced.

According to another embodiment, the pressure receiving portion and / or at least one protrusion thereof is present on at least half of the longitudinal length of the closing element in an insertion direction of the fluid line into the closing element.

According to another embodiment of the invention, the pressure receiving portion comprises a plurality of protrusions arranged at different positions along the longitudinal length of the closing element in an insertion direction of the fluid line into the main body.

For example, the closing portion may comprise at least two plates of different thicknesses that are arranged on the main body of the closing element. Due to the different thicknesses of the plates of the closing portion, the protrusions arranged on the plates are positioned at different positions along the longitudinal length of the closing element in an insertion direction of the fluid line into the main body.

According to another embodiment of the invention, the at least one protrusion of the pressure receiving portion is configured in a hook shape.

Additionally or alternatively, adjacent to the at least one protrusion of the pressure receiving portion a recess is formed. The recess may also define the protrusion as the protrusion may not extend above the other areas of the plate with the exception of the area of the recess.

According to an embodiment of the invention, the pressure receiving portion and / or at least one protrusion thereof at least partially defines a distal end face of the closing element in the insertion direction of the fluid line into the closing element.

This may have the advantage that the distance a pressure exertion element to be brought into contact with the pressure receiving portion and / or at least one protrusion thereof by a relative movement of the pressure exertion element and the closing element has to travel before such contact is established is reduced and/or minimized.

Alternatively, the closing element or a connector comprising a fluid line the closing element is arranged on may be moved relative to the pressure exertion element. In this case, the distance the closing element and / or connector has to travel to establish contact to the pressure exertion element may be reduced and/or minimized.

According to an embodiment of the invention, the main body has a cylindrical or polygonal, in particular a hexagonal, form.

As it may be beneficial, the main body may have a polygonal shape and the number of plates forming the closing portion corresponds to the number of edges of the polygonal main body, in particular, wherein the main body has a hexagonal form and the closing portion comprises six plates. This ensures that each plate forming the closing portion can be arranged on a straight edge of the main body which facilitates tilting of the plates, because leverage is optimized.

According to another embodiment of the invention, at a connection point between the closing portion comprising the intended breaking point and the main body of the closing element the material is selectively weakened. The weakening may be realized by providing at least one recess, to facilitate breaking of the intended breaking point due to pressure being applied to the pressure receiving portion. Alternatively or in combination the weakening may be achieved by using a less stable and/or more flexible material and/or by providing a hollow structure.

For example, the closing portion may comprise a plurality (e.g. six) plates having the shape of circle sections that are arranged around a central point similar to petals of a flower. These plates are may be connected, i.e. joined, to a hexagonal main body. At the connection point between the main body and the plates, the material can be weakened, e.g. by providing an annular recess running long the outer circumference of the main body.

According to another embodiment, the closing element further comprises a sealing structure at the inner circumference of the main body, in particular in the form of an annular protrusion running along the entire circumference of the main body. Alternatively, the sealing function may be provided by a flush fit between the inner circumference of the main body of the closing element and an outer circumference of a fluid line inserted into the main body.

The sealing structure may be configured to be received in a corresponding annular recess running along the outer circumference of a fluid line to removably hold the closing element to the fluid line.

Furthermore, the closing element may comprise a recess adjacent to the sealing structure arranged in a position radially outwards from the sealing structure to allow radial movement of the sealing structure. This ensures easy mounting of the closing element on a fluid line.

According to an embodiment of the invention, the closing element is at least partially or completely manufactured from polymer material, in particular from linear low density polyethylene and / or high density polyethylene.

The closing element may be manufactured by injection moulding.

Another aspect of the invention relates to a connector, in particular a connector of a concentrate container or other disposable, comprising at least one fluid line providing a flow path through the connector via that fluid may be passed into the container to dissolve concentrate and / or fluid may be withdrawn from the container, wherein a closing element according to the present invention is arranged on at least one fluid line of the connector to close the fluid line. The closing effect may be a closing effect to block fluid from exiting and/or entering the connector.

The at least one fluid line of the connector can comprise only one lumen (only one tube or hose) or can comprise an inner lumen and an outer lumen (a tube in a tube or hose in a hose). The outer lumen may not provide a flow path for conducting fluid through the connector and may be configured as an annular blind hole.

If the fluid line of the connector comprises only one lumen, the closing element according to the present invention may be arranged on this lumen / tube.

If the fluid line of the connector comprises an inner lumen and an outer lumen, the closing element may be arranged on an end face of the inner lumen. An end face of the inner lumen may be arranged retracted into the outer lumen so that a gap is present between the end face of the inner lumen and the end face of the outer lumen. This may support that the inner lumen is not brought into contact with the closing element.

If the fluid line of the connector comprises an inner lumen and an outer lumen, the closing element may be arranged on an end face of the outer lumen and an end face of the inner lumen may be arranged retracted into the outer lumen so that a gap is present between the end face of the inner lumen and the closing element. This may support that the inner lumen is not brought into contact with the closing element.

The outer lumen may not run through the connector to provide a flow path therethrough. Only the inner lumen may be used to conduct fluid through the connector and the other lumen may serve the purpose of preventing the closing element from coming into contact with the inner lumen and/or may serve the purpose that the outer surface of the inner lumen may not be touched by a user.

According to another embodiment of the invention, the connector comprises at least two fluid lines, each closed, for example fluidically closed, by a closing element according to the present invention. The two fluid lines may have different lengths, so that a pressure exertion element being moved towards the connector reaches the at least two fluid lines sequentially. Thus, the at least two fluid lines may be sequentially opened by the pressure exertion element.

At least one of the fluid lines of the connector may comprise a sharp or pointed leading edge pointing away from the connector. In other words, the sharp or leading edge is pointing towards a fluid line that is to be connected to the fluid line. This may support the opening process.

According to an embodiment of the invention, at least one of the fluid lines of the connector comprises an annular recess at its outer circumference to receive a sealing structure of a closing element to thereby hold the closing element to the fluid line.

Another aspect of the present invention relates to a system comprising a closing element according to the present invention, a connector according to the present invention and a pressure exertion element, in particular a connector element of a blood treatment device or a dosing unit thereof, wherein the pressure exertion element is configured to be fluidically connected to the fluid line of the connector by breaking the intended breaking point of the closing element, in particular by a relative movement of the pressure exertion element and the connector.

The pressure exertion element may be moved towards the connector comprising the closing element and / or the connector comprising the closing element may be manually or automatically moved towards the pressure exertion element.

According to an embodiment of the invention, the connector, in particular the connector of a concentrate container, and / or the pressure exertion element, in particular the connector element of a blood treatment device or a dosing unit thereof, comprises at least one fluid line comprising an inner lumen and an outer lumen. The inner lumen and the outer lumen may be arranged concentrically to each other.

The inner lumen may be arranged retracted into the outer lumen. In this embodiment the inner lumen is used to conduct fluid and the closing element is arranged on the outer lumen that does not constitute a flow path through the connector but serves the purpose to ensure that the closing element is not brought into contact with the inner lumen. Alternatively, the closing element may be arranged on the inner lumen. The inner lumen may be arranged retracted in the outer lumen. Further, alternatively only one tube or hose forming a lumen may be present that is used for conducting fluid and is closed by the closing element.

The embodiments of the closing elements disclosed herein purport only to illustrate but not limit the present invention and modifications of the embodiments disclosed herein are well within the scope of the present invention. For example, the shape and number of the at least one protrusion or protrusions of the pressure receiving portion can be adapted to any specific desired application.

If a container comprising such a closing element is to be connected to a blood treatment device, the connector element of the blood treatment device is moved in such a way as to come into contact with and exert pressure on the pressure receiving portion of the closing element. In this case the connector element of the blood treatment device functions as a pressure exertion element. The connector element may be moved towards the connector of the container and thus the fluid line closed by the closing element by linear movement or a tilting movement, e.g. if the connector element of the blood treatment device is arranged on a movable flap of the blood treatment device. For example, the connector element may have an outer lumen that receives the closing element and abuts / contacts the pressure receiving portion thereof. Alternatively, the connector bearing the closing element may be moved towards the connector element of the blood treatment device.

The closing element, that may have a shape of a cap, is pressed down onto the fluid line until the intended breaking point of the closing portion breaks and the fluid line is fluidically opened.

According to an aspect of the invention, the pressure receiving portion comprises an annular protrusion running at least along a part of or along the entire length of the outer circumference of the closing element, in particular of the pressure receiving portion thereof.

Alternatively or additionally, the pressure receiving portion may comprise a plurality of protrusions arranged along the outer circumference of the closing element, wherein the protrusions may be separate from another and / or arranged in equal or different intervals along the outer circumference.

Providing separate protrusions may support efficient force transfer from the pressure receiving portion to the intended breaking point, especially if the closing portion comprises several plates arranged around a central point like petals of a flower and an intended breaking point, e.g. a line of thinner material is present between each two plates or petals.

This may have the advantage that if the connector element of the blood treatment device is moved downwards onto the closing element to contact the pressure receiving portion and press the closing element down onto the fluid line until the intended breaking point opens, the connector element of the blood treatment device has to be moved a shorter distance before it abuts the pressure receiving portion. Thus, less space may be required for the components for fluidically coupling the concentrate container to the blood treatment device or dosing unit allowing a more compact design of the dosing unit.

In order to reduce the distance the connector element of the blood treatment device has to travel to establish contact with the pressure receiving portion, the pressure receiving portion and / or at least one protrusion thereof can be arranged to define a distal end face of the closing element in the insertion direction of the fluid line into the closing element. In other words, the plane of the upper end face of the closing element or cap may be at least partially be defined by the pressure receiving portion when the closing element is arranged on a fluid line to fluidically close that fluid line.

As it may be beneficial, the pressure receiving portion may comprise a plurality of protrusions arranged at different positions along the longitudinal length of the closing element in an insertion direction of the fluid line into the closing element. In this case, if the connector element of the blood treatment device is moved down onto the closing element, the protrusions arranged at different longitudinal positions are sequentially opened (starting with the top protrusion) as the connector element moves from the upper end of the closing element to the lower end of the closing element. For example, in the case of the closing portion comprising several plates arranged like petals of a flower separated from each other via intended breaking points, one plate or multiple plates can have different thicknesses, so that the pressure exertion element sequentially abuts the plates of different thicknesses. The thickness of the plates can be varied depending on the specific application, for example, only one of the plates of the closing portion can have a thickness that differs from the thickness of the other plates that have a uniform thickness. Alternatively, two or more plates may have differing thicknesses from the rest of the plates. Thus, the flow diameter of the opening of the concentrate container can be sequentially increased in a step-wise fashion and the maximal force required for opening the closing element can be reduced.

According to embodiments of the invention, the closing element comprises a cylindrical or hexagonal main body for receiving an end portion of the fluid line. Other geometries of the main body, e.g. a heptagonal, triangular or quadratic base area, are possible, in particular as long as the closing element can still be arranged on a fluid line to be closed in the fashion of a cap.

If a hexagonal main body is used in conjunction with a closing portion comprising six plates, this offers the possibility that each of the six plates of the closing portion can be arranged on a straight edge of the hexagonal main body. This facilitates opening of the closing element upon the exertion of pressure on to the pressure receiving portion due to the straight edges supporting the tilting motion of the plates of the closing portion. The plates of the closing portion act as levers that break the intended breaking point. The configuration of the intended breaking point as straight lines arranged between the plates may optimize leverage.

The closing element may comprise a main body configured to receive the fluid line to be closed and a closing portion configured to fluidically close the fluid line.

To facilitate a breaking of the intended breaking point by the connector element and thus ensure an easy and reliable opening of the container, at least one connection point between the closing portion comprising the intended breaking point and the main body of the closing element the material may be selectively weakened, for example by providing at least one recess.

In this embodiment, the closing portion and the pressure receiving portion may be integrally formed so that upon the exertion of pressure onto the pressure receiving portion the closing portion or at least a part thereof is moved by a tilting motion to break the intended breaking point.

The weakening of the material at the at least one connection point between the closing portion comprising the intended breaking point and / or the pressure receiving portion and the main body of the closing element allows the closing portion and / or the pressure receiving portion to easily tilt and thereby break the intended breaking point. This is especially relevant in the case of the closing portion comprising multiple plates arranged like petals of a flower around a central point, wherein the plates are separated from each other by lines of weakened material forming intended breaking points.

To improve the storage ability of a container closed by a closing element according to the present invention, the closing element may further comprise a sealing structure at the inner circumference of the main body, for example in the form of an annular protrusion running along the entire circumference of the main body, to ensure an air-tight sealing of the container.

The closing element may be at least partially or completely manufactured from polymer material, in particular from linear low density polyethylene and / or high density polyethylene. These materials offer an optimal tradeoff between mechanical stability rigidity and thus reliable breaking of the intended breaking point and compliance ensuring the effort required for breaking the closing element remains reasonable. Elastomers are also possible, either alone or in combination with linear low density polyethylene and / or high density polyethylene.

Another aspect of the invention relates to a connector, in particular a connector of a concentrate container or other disposable, wherein a closing element according to the present invention is fixedly or removably arranged on at least one fluid line of the connector to for example fluidically close the fluid line.

The fluid line may provide a flow path through the connector and can e.g. be used to conduct fluid into the concentrate container or to withdraw liquid concentrate from the container. The fluid line may be any tube or hose.

The connector of the disposable may comprise at least one holding element, e.g. in the shape of a hook configured to hold the connector in the vicinity of a connector element of a blood treatment device or dosing until thereof to that the connector of the disposable shall be connected.

The connector of the disposable may also comprise a structure for opening a valve of the connector element of the blood treatment machine or dosing unit thereof upon connection of the connector of the disposable to the connector element of blood treatment machine or dosing unit thereof. For example, a fluid line of the connector of the disposable may comprise a central pole or spike configured to remove a valve element of its valve seat in a valve of the blood treatment machine or dosing unit thereof to thereby enable fluid flow through the valve. The shape of the pole or spike is arbitrary, any structure suitable for opening the valve upon connector of the connector to the connector element may be chosen.

The fluid line of the connector may comprise an inner lumen or inner tube and an outer lumen or outer tube, wherein the closing element is arranged on an end face of the outer lumen and an end face of the inner lumen is arranged retracted into the outer lumen so that a gap is present between the end face of the inner lumen and the closing element. In other words, the outer tube serves to protect or shield the inner lumen from contact with the closing element. This may offer the advantage that contamination of the inner tube that is used to conduct fluid is reduced. According to an alternative embodiment, the closing element may be present on the end face of the inner lumen, as indicated for example in Fig. 17.

According to an embodiment, the connector comprises at least two fluid lines, each closed by a closing element according to the present invention, wherein the two fluid lines project different lengths from an end face of the connector.

If a concentrate container comprising such a connector is to be connected to a blood treatment device or dosing unit, the two fluid lines may be sequentially opened and connected to the blood treatment device or dosing unit, because first, the fluid line projecting further from the connector towards the blood treatment device or dosing unit is opened and secondly the shorter fluid line is opened and connected.

Yet another aspect of the invention relates to a system comprising a closing element according to the present invention, a connector (for example of a concentrate container) according to the present invention and at least one connector element, for example a connector element of a blood treatment device or a dosing unit thereof, wherein the connector element is configured to be fluidically connected to at least one fluid line of the connector by breaking the intended breaking point of the closing element arranged thereon.

As it may be beneficial, the connector, in particular a connector element of a blood treatment device or a dosing unit thereof, may comprise an inner lumen and an outer lumen for example arranged concentrically to each other. Any lumen may have the form of a tube or hose.

The connector element of the blood treatment machine or dosing unit thereof may be moved towards and / or onto the connector of the disposable and its fluid lines by a translational or linear movement, or by a rotational movement in that for example, the connector element is arranged in a flap of the blood treatment device that is tilted to come into contact with the connector of the disposable.

As it may be beneficial, the connector element of the blood treatment machine may be configured movable relatively to a main body of the blood treatment device, so that it can be selectively moved to establish a connection to the disposable.

For example, the connector element of the blood treatment device or dosing unit may comprise a portion that is movable relative to the main body of the device for establishing a fluid connection between the disposable and the blood treatment device and another portion, that may be fixedly arranged at the main body of the device, for holding the connector of the disposable in place.

The movable portion may contains the fluid lines of the blood treatment device and / or is arranged further up on the main body of the blood treatment device, if the blood treatment device is placed onto a horizontal plane in its normal operation position. The movable portion containing the fluid lines may be moved towards the connector by a translational or rotational movement.

Further features, effects and advantages of the present invention become apparent from the subsequent detailed description of embodiments of the invention under reference to the appended drawings. The same or similar components are indicated by the same reference signs. In the drawings:
Fig.1 shows a perspective view of a closing element according to an embodiment of the invention;
Fig.2 shows a sectional view of the closing element of Fig. 1;
Fig.3 shows a first step in the connection process of a connector and closing element of Fig. 1 being connected to a connector element of a blood treatment device;
Fig. 4 shows a second step in the connection process of a connector and closing element of Fig. 1 being connected to a connector element of a blood treatment device;
Fig.5 shows a third step in the connection process of a connector and closing element of Fig. 1 being connected to a connector element of a blood treatment device;
Fig.6 shows a fourth step in the connection process of a connector and closing element of Fig. 1 being connected to a connector element of a blood treatment device;
Fig.7 shows sectional view of a closing element according to another embodiment;
Fig.8 illustrates the sequential opening of two fluid lines each closed by a closing element according to the present invention;
Fig.9 shows sectional view of a closing element according to yet another embodiment.
Fig. 10a shows a machine connector of a blood treatment machine that is movable by rotation in a closed state;
Fig. 10b shows the machine connector of Fig. 10a in an open state;
Fig. 11 shows a different machine connector of a blood treatment machine that is translationally movable;
Fig. 12 shows a connector of a concentrate container attached to a blood treatment device;
Fig. 13 shows a perspective view of a closing element according to another embodiment of the invention;
Fig. 14 shows a bottom view of the closing element of Fig. 13;
Fig. 15 shows an overview of exemplary dimensions in mm of an embodiment of a closing element according to the disclosure, for example of the embodiment of Fig. 13;
Fig. 16 illustrates the reduction of force required to open a closing element with the dimensions as shown in Fig. 15; and
Fig. 17 shows a connector with a closing element as shown in Fig. 13.

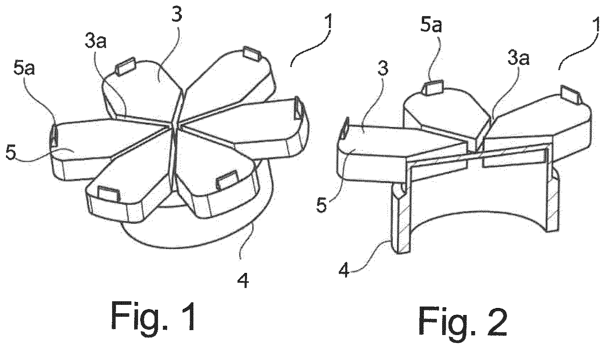

Fig.1 shows a perspective view of a closing element 1 according to an embodiment.

The closing element comprises a main body 4 and in this embodiment six elements or plates movably joined to the main body 4 that together form the closing portion 3 and the pressure receiving portion 5 of the closing element. These elements may be arranged in the fashion of flower petals. Each plate may have the shape of a section of a circle. The outer edges of each plate in the radial direction may be arranged in a rectangular shape.

At its outer edge, each of these plates may be equipped with a protrusion 5a that is part of the pressure receiving portion 5 and may be configured as a hook to hold e.g. an outer tube 10 of a connector element 9 of a blood treatment device. Each plate or petal, in particular the pressure receiving portion thereof, projects outwards over the main body 4 of the closing element 1, so that upon the exertion of pressure thereon, each plate can easily tilt. The plates thus act as levers.

The elements forming the plates or petals of the flower-shape are separated from each other by intended breaking points 3a. In conjunction with the design of the intended breaking point and the shape of the plates, the provision of separate protrusions may have the effect that the force and strain acting on the intended breaking points upon the exertion of pressure on the plates is optimized.

Fig.2 shows a sectional view of the closing element of Fig. 1.

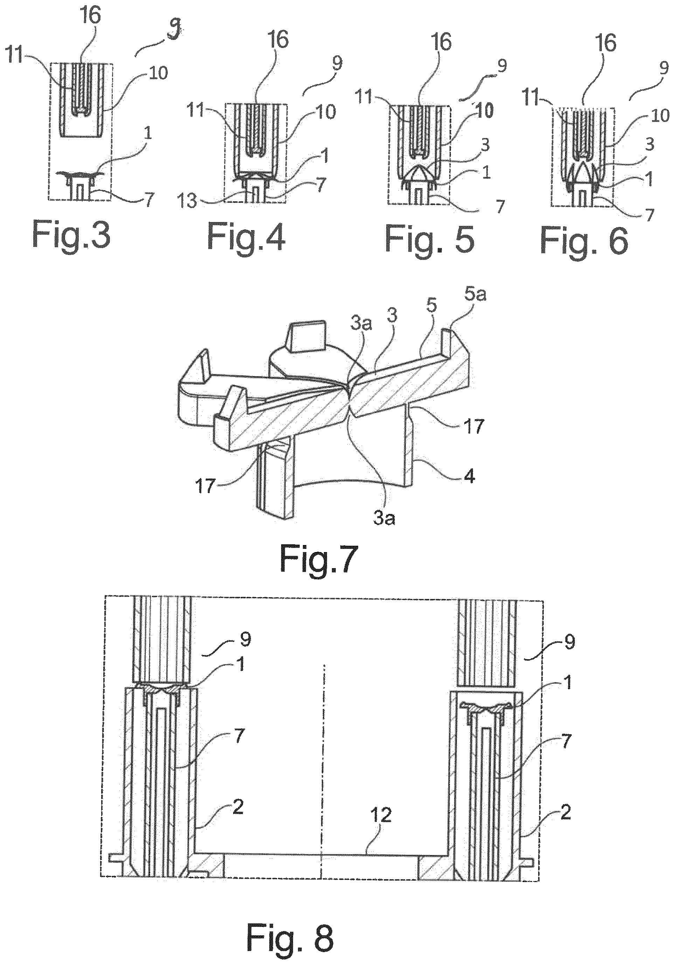

Fig.3 shows a first step in the connection process of a tube 7 closed by a closing element 1 of Fig. 1 being connected to a connector element 9 of a blood treatment device.

At the stage shown in Fig. 3, the connector element 9 and the tube 7 comprising the closing element 1 are aligned to each other.

At the stage shown in Fig.4, the connector element 9 has been moved towards the closing element 1 so that the leading edge of the outer tube 10 abuts the pressure receiving portion 5 of the closing element 1.

At the stage shown in Fig.5, the connector element 9 has been moved further onto the tube 7 and presses onto the pressure receiving portion 5 of the closing element 1 so that the closing portion 3 of the closing element and thus the intended breaking point 3a thereof is strained due to the tilting or rotating movement of the elements ("petals") forming the closing portion 3. The edge of the tube 7 presses against the elements forming the closing portion from the inside of the closing element 1 to facilitate the opening thereof.

At the stage shown in Fig.6, the connector element 9 has been moved so far onto the tube 7 that the pressure applied on the pressure receiving portion 5 of the closing element 1 exceeds the force required to break the intended breaking point 3a that is broken in Fig. 6. As shown in Fig.60, the elements ("petals") forming the closing portion 3 have tilted or rotated to thereby open the closing element 1.

Thus, in this embodiment, the closing element 1 is opened by a tilting or rotating movement of the elements of the closing portion that act as levers.

Fig.7 shows sectional view of a closing element 1 according to another embodiment. In this embodiment, the material at a connection point 17 between the elements ("petals") forming the closing portion 3 and pressure receiving portion 5 and the main body 4 of the closing element 1 has been selectively weakened to ensure easy tilting / rotating of the elements ("petals") forming the closing portion 3 and pressure receiving portion 5 upon the application of pressure thereon.

The weakening of the material may involve the removal of up to 50% of the material, in particular up to 30% or up to 20 % of the material. Depending on the material, a removal of up to 80 % of the material may be realized.

Fig.8 illustrates the sequential opening of two fluid lines, in this case the inner tubes 7 thereof, each closed by a closing element 1 according to the present invention. The inner tube 7 arranged to the left in Fig. 8 projects further away from the connector 12 and thus is closer to the connector element 9 of the blood treatment device. As the connector element 9 of the blood treatment device is moved towards the connector 12, the tube 2 on the left is reached by the connector element 9 first and thus is also opened first.

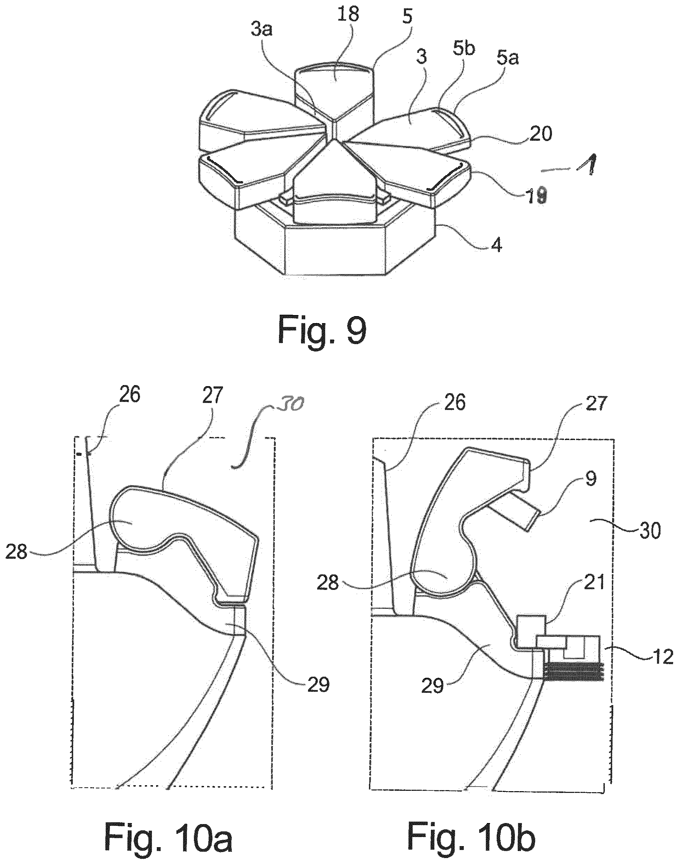

Fig.9 shows sectional view of a closing element 1 according to yet another embodiment. In this embodiment, the main body 4 of the closing element 1 has a hexagonal shape. The closing portion 3 and the pressure receiving portion 5 are formed by six elements arranged like the petals of a flower.

In this embodiment, each protrusion 5a of the pressure receiving portion 5 of each petal is formed by a grove or recess 5b. The protrusion 5a protrudes relative to the surface defined by the grove 5a.

As illustrated in Fig. 9, the protrusions 5a of the pressure receiving portion 5 and / or the elements forming the closing portion 3 and / or the pressure receiving portion 5 may be arranged at different positions along the longitudinal direction of the closing element 1.

For example, in Fig. 9 the element arranged top most in the longitudinal direction is indicated by reference numeral 18. One of the four elements arranged second from the top in the longitudinal direction is indicated by reference numeral 19. The element indicated by reference numeral 20 is arranged lowest in the longitudinal direction of the closing element 1. Thus, the element 18 has a larger thickness than the elements 19 and the element 20 has the smallest thickness. All elements 18, 19, 20 forming the closing portion 3 are attached to the hexagonal main body 4 in the same longitudinal position, e.g. on a common continuous edge of the hexagonal main body. The elements are plates in this embodiment.

Thus, if a connector element 9 of a blood treatment device is moved onto the closing element 1 coming from the top in Fig. 9, the leading edge of the outer tube 10 of the connector element 9 first hits the top most element 18, then hits the intermediate elements 19 and finally hits the lowest element 20. Thus, the flow cross section of the closing element is sequentially enlarged in a step-wise fashion. In addition to that, the force required for opening the closing element can be reduced.

Fig. 10a shows a machine connector 30 of a blood treatment device 26 comprising a first part 27 that is movable by rotation around a hinge 28 and a second part 29. In the state shown in Fig. 10a, the connector element 26 is closed so that the first part 27 abuts the second part 29 that is unmovably affixed at the blood treatment device 26.

The first part 27 comprises two connector elements 9 in the shape of fluid lines for fluidically connecting the blood treatment device 26 e.g. to a concentrate container. The connector elements 9 can have the configuration shown in Fig. 3-6. The first part 27 can have the shape of a cap or flap that can be moved via the hinge 28. The movement of the first part 27 can be achieved manually or automatically via a motor. The movement of the first part 27 opens the closing element(s) 1 present on the connector 12 and establishes a fluid connection between the blood treatment device 26 and the connector 12.

Fig. 10b shows the machine connector 30 of Fig. 10a in an open state. A connector 12 has been affixed to the second part 29. The second part 29 comprises two recesses (not visible in Fig. 10b) into that the two attachment elements 21 of the connector 12 have been inserted. The first part 27 comprises two connector elements 9, i.e. fluid lines that each can be inserted into a recess of the second part 29 for rinsing or can be used to establish a fluid connection to a concentrate container via the connector 12.

The connector elements 9 (only one visible in Fig. 10) / fluid lines of the first part of the machine connector 30 of the blood treatment device 26 may each comprise an inner tube 11 and an outer tube 10 and thus contain two lumina. The inner tube 11 serves the purpose of conducting fluid and is arranged retracted within the outer tube 10. The outer tube 10 is used e.g. for exerting pressure onto a closing element 1. After the closing element 1 has been opened by the outer tube 10, the inner tube 11 is inserted into the fluid line (e.g. the tube 7) of the connector 12 to fludicially connect the blood treatment device 26 and the connector 12 and the concentrate container attached thereto. Thus, the sterile inner tube 11 is never brought into contact with the non-sterile closing element 1.

In the lumen of the inner tube 11 a valve comprising a valve element 16 may be arranged. The fluid line of the connector 12, e.g. the tube 7, comprises a means 13, e.g. a central pole, for opening the valve by displacing the valve element 16. Thus, the valve is opened only after the connection of the connector 12 to the machine connector 30 of the blood treatment device. Thus, spilling of fluid from the connector elements 9 or fluid lines is avoided.

To connect the blood treatment device to the concentrate container, the first part 27 of the machine connector 30 is moved downwards towards the connector 12 until the fluid line / connector element 9 is inserted therein and opens a closing element present on the fluid line / tube of the connector 12.

The connector 12 is affixed to the second part 29. The second part 29 comprises attachment means to that the connector 12 may be affixed via its attachment means 21. The attachment means of the second part 29 can be for example recesses (such as the recesses 31) or protrusions (such as the protrusions 32). These attachment means may be fixedly and non-movably connected to the blood treatment device 26.

Between treatment sessions, the connector elements 9 of the blood treatment device 26 need to be rinsed or disinfected. For this purpose, the connector elements 9of the first part 27 are inserted into the recesses 31 of the second part 29 until the lumen of the outer tube 10 is fluidically closed. The inner tube 11 is arranged retracted into the lumen of the outer tube 10 and thus can be rinsed in this position, because the connector element 9 is fluidically short-circuited.

In this case, rinsing fluid is provided via the lumen of the inner tube 11 and removed to a drain via the lumen of the outer tube 10. Each rinsing recess 31 may be equipped with a structure for actuating a valve to allow the flow of rinsing fluid. These valve actuators can have the shape of central poles 34 (see Fig. 26). In addition to that, the rinsing recesses 31 can be equipped with a drain to remove remaining rinsing fluid.

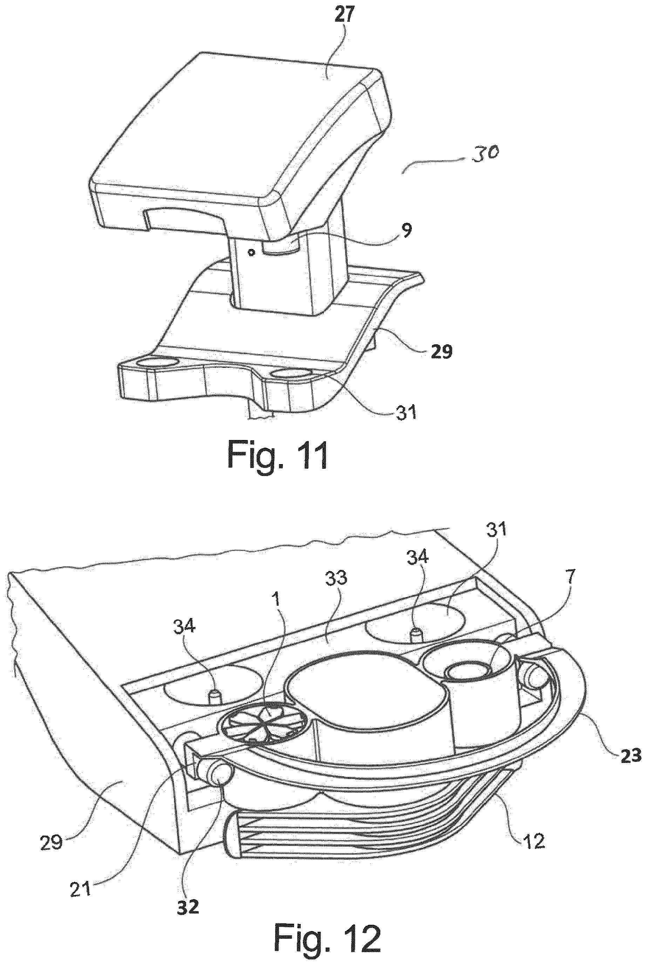

Fig. 11 shows a different machine connector 30 of a blood treatment device 26 the first part 27 thereof is translationally movable. In Fig. 25 the rinsing recesses 31 in the second part 29 of the machine connector 30 can be seen. A connector 12 can be affixed to the second part 29 by insertion of the attachment elements 21 into the rinsing recesses 31 and the first part 27 comprising the connector element 9 is subsequently moved downwards to establish a fluid connection between the blood treatment device and the concentrate container through the connector 12.

Fig. 12 shows a connector 12 of a concentrate container attached to a blood treatment device 26 via its attachment elements 21. The second part 29 of the machine connector of the blood treatment device 26 comprises a movable drawer 33 comprising the rinsing recesses 31. It is also possible to arrange the rinsing recesses 31 in a fixed position relative to the blood treatment device 26. The movable drawer 33 further comprises attachment elements 32 that are pole-shaped.

The connector 12 comprises attachment elements 21 that have the shape of curved arms configured to receive the corresponding attachment elements 32 of the blood treatment device that are pole-shaped. In the embodiment shown in Fig. 26 the connector is thus not affixed to the blood treatment device via the rinsing recesses 31 but via separate attachment elements 32 and 21 that interact with each other.

Upon connection of the connector 12 to the movable drawer 33 of the second part 29, the movable drawer 33 is then moved towards the blood treatment device 26.

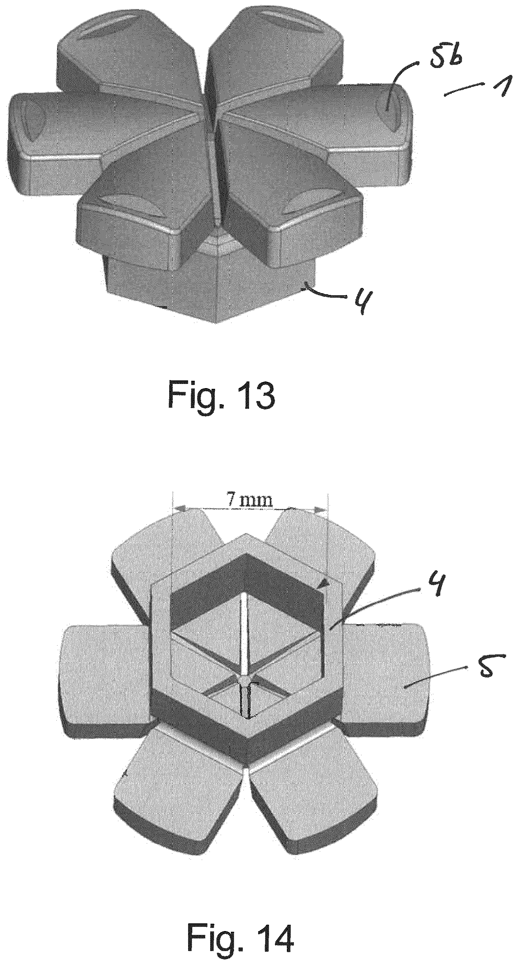

Fig. 13 shows a perspective view of a closing element according to another embodiment of the invention, in this embodiment, the closing element has a main body 4 of a hexagonal form and six plates arranged in the form of petals that form the closing portion and the pressure receiving portion of the closing element 1.

Fig. 14 shows a bottom view of the closing element of Fig. 13; in this embodiment, the distance between two opposing inner side surfaces of the hexagonal main body 4 is about 7 mm.

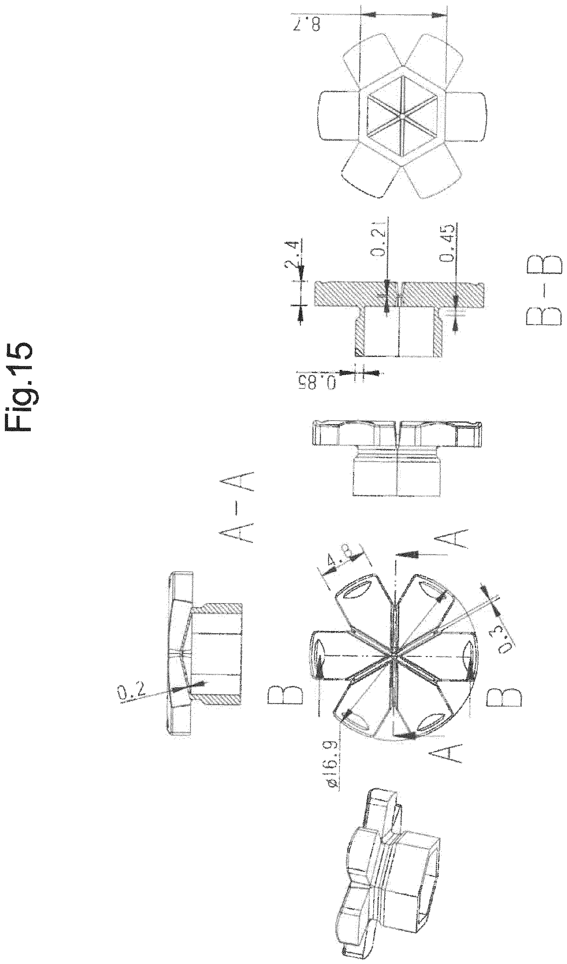

Fig. 15 shows an overview of an exemplary embodiment of the closing element as described with to reference to Fig. 13; the numbers as identified in Fig, 15 are dimensions in mm and were carefully chosen to reduce the amount of force required to open the closing element 1.

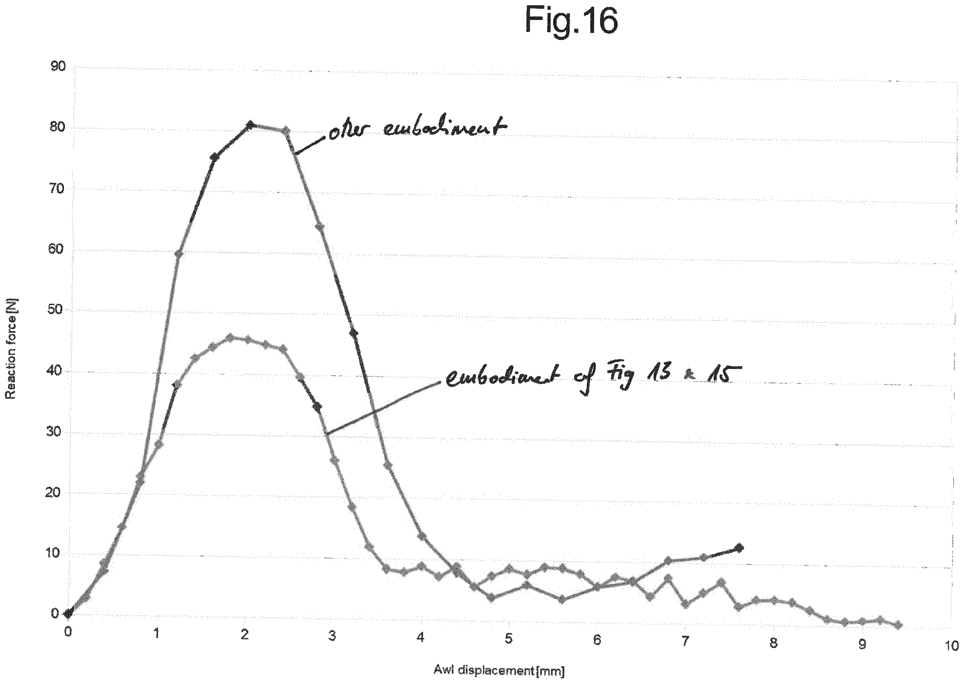

Fig. 16 illustrates the reduction of force required to open a closing element with the geometry and dimensions as shown in Fig. 13 and Fig. 15 compared to an embodiment of the invention with other dimensions. By carefully choosing the dimensions, the force required could be reduced to 46N instead of 81N.

Fig. 17 shows a connector with a closing element as shown in Fig. 13. The closing element may be arranged on an inner lumen 7 of a fluid line and closes the fluid line.

An outer lumen 2 surrounds the inner lumen 7. A connector element 9 of a blood treatment device may be moved downwards to press onto the closing element 1 and open it. The inner lumen 7 has an outer shape being hexagonal in correspondence with the hexagonal inner shape of the main body of the connector element 1. The dimensions of the inner lumen 7 are such that the inner lumen 7 fits into the main body of the connector element 1.

The inner lumen 7 comprises a champfer C at its leading edge. The champfer C is present on a radially outward surface of the inner lumen 7 and is inclined with an angle of 45 ° in this example.

## Claims

1. Closing element for a fluid line, in particular a fluid line of a concentrate container, comprising:
a main body configured to at least partially receive the fluid line,
a closing portion configured to be arranged on an open portion of the fluid line to close the fluid line, wherein the closing portion comprises a plurality of plates arranged around a common center point and each separated from another over an intended breaking point, and
a pressure receiving portion configured to receive pressure applied by a pressure exertion element, in particular a connector element to be fluidically connected to the fluid line by breaking the intended breaking point, wherein
the pressure receiving portion is formed by that part of the plurality of plates projecting outwards from the closing portion or the main body.

2. Closing element according to claim 1, wherein each plate is arranged on the main body so that a length of a part of each plate projecting outwards from the main body is at least 80% of, in particular the same or in particular larger than, a length of a part of each plate projecting inwards from the main body.

3. Closing element according to claim 1 or 2, wherein a length from an outer edge of a first plate of the plurality of plates to an outer edge of a second plate of the plurality of plates arranged opposed to the first plate lies in the range of between 10 mm and 20 mm, in particular between 12 mm and 18 mm, in particular between 16 mm and 17 mm.

4. Closing element according to one of the preceding claims, wherein a width of an intended breaking point separating a first plate of the plurality of plates from a second plate of the plurality of plates lies in the range of between 0,1 mm and 0,5 mm, in particular between 0,2 mm and 0,4 mm, in particular between 0,25 mm and 0,35 mm.

5. Closing element according to one of the preceding claims, wherein the pressure receiving portion comprises a plurality of protrusions arranged along the outer circumference of the closing element, in particular at the pressure receiving portion thereof, wherein the protrusions are in particular separate from another and / or arranged in equal intervals along the outer circumference.

6. Closing element according to claim 5, wherein each protrusion of the plurality of protrusions is arranged adjacent to an outer edge of a plate of the plurality of plates so that pressure applied to each protrusion is selectively transferred to the plate the protrusion is arranged on, wherein a length from a protrusion of a first plate of the plurality of plates to protrusion of a second plate of the plurality of plates arranged opposed to the first plate lies in the range of between 10 mm and 20 mm, in particular between 12 mm and 18 mm, in particular between 16 mm and 16,6 mm.

7. Closing element according to one of the preceding claims, wherein the pressure receiving portion and / or at least one protrusion thereof at least partially defines a distal end face of the closing element in the insertion direction of the fluid line into the closing element.

8. Closing element according to one of the preceding claims, wherein the main body has a hexagonal form, wherein a distance between a first outer side surface of the hexagon and a second outer side surface of the hexagon arranged opposed to the first outer side surface lies in the range of between 6 mm and 10 mm, in particular between 7,5 mm and 9,5 mm, in particular between 8 mm and 9 mm.

9. Closing element according to one of the preceding claims, wherein at a connection point between the closing portion comprising the intended breaking point and the main body of the closing element the material is selectively weakened, in particular by providing at least one annular recess running along the circumference of the main body, to facilitate breaking of the intended breaking point due to pressure being applied to the pressure receiving portion, wherein a width of the annular recess lies in the range of between 0,3 mm and 0,6 mm, in particular in the range between 0, 35 mm and 0,55 mm, in particular between 0,4 mm and 0,5 mm.

10. Closing element according to one of the preceding claims, wherein the closing element is at least partially or completely manufactured from polymer material, in particular from linear low density polyethylene and / or high density polyethylene.

11. Connector, in particular connector of a concentrate container or other disposable, comprising at least one fluid line providing a flow path through the connector, wherein a closing element according to one of the preceding claims is arranged on at least one fluid line of the connector to in particular fluidically close the fluid line.

12. Connector according to claim 11, wherein the fluid line comprises an inner lumen and an outer lumen, wherein the inner lumen in particular has a hexagonal form and the closing element is arranged on an end face of the inner lumen, wherein the end face of the inner lumen comprises a champfer, in particular at its leading edge.

13. Connector according to claim 12, wherein the champfer of the leading edge of the inner lumen is present on a radially outward side of the inner lumen and the champfer comprises a tapered portion inclined with an angle of between 35 ° to 65°, in particular between 40° and 50°, in particular between 42° and 47°.

14. System comprising a closing element according to one of claims 1 to 10, a connector according to one of claims 11 to 13 and a pressure exertion element, in particular a connector element of a blood treatment device or a dosing unit thereof, wherein the pressure exertion element is configured to be fluidically connected to the fluid line of the connector by breaking the intended breaking point of the closing element, in particular by a relative movement of the pressure exertion element and the connector.
